# EUROPÄISCHE PATENTSCHRIFT

(11) **EP 1 536 753 B1**
(45) Veröffentlichungstag und Bekanntmachung des Hinweises auf die Patenterteilung: **17.02.2016**
(21) Anmeldenummer: 03798892.0
(22) Anmeldetag: 04.09.2003
(51) Int. Cl.: A61K 38/39

(54) **VERFAHREN ZUM SCHUTZ UND ZUR MODULATION VON DERMAL EPIDERMAL JUNCTIONS**
METHOD FOR PROTECTING AND FOR MODULATING DERMAL-EPIDERMAL JUNCTIONS
PROCEDE DE PROTECTION ET DE MODULATION DES JONCTIONS DERME-EPIDERME

(30) Priorität: 13.09.2002 EP 02292247
(43) Veröffentlichungstag der Anmeldung: 08.06.2005
(73) Patentinhaber: BASF Beauty Care Solutions France S.A.S., 69007 Lyon (FR)
(72) Erfinder: JEANMAIRE, Christine, F-54000 Nancy (FR); PAULY, Gilles, F-54000 Nancy (FR)
(74) Vertreter: Reinhardt, Jürgen
(86) Internationale Anmeldenummer: PCT/EP2003/009809
(87) Internationale Veröffentlichungsnummer: WO 2004/030639

(56) Entgegenhaltungen:
- WO-A-90/13302
- WO-A-96/40047
- WO-A-99/62480
- WO-A-99/62481
- DE-A- 19 950 020

## Beschreibung

### Gebiet der Erfindung

Die Erfindung befindet sich auf dem Gebiet der kosmetischen Zubereitungen und betrifft ein kosmetisches Verfahren zur Verbesserung und/oder zum Schutz der Dermal-Epidermal Junctions der Haut, Kopfhaut und Schleimhaut und zum Schutz der menschlichen Haut gegen die Alterung, oxidativen Stress und gegen schädigende Einflüsse durch Umweltgifte und UV-Strahlung. Des weiteren betrifft die Erfindung die Verwendung einer Substanz, welche eine Modulation von Plectin/HD1 und/oder Entactin/Nidogen und/oder Perlecan bewirkt, zur Herstellung kosmetischer Mittel zur Verbesserung und/oder zum Schutz der Dermal Epidermal Junctions.

### Stand der Technik

Die Basalmembran ist eine verbindende Zellstruktur zwischen morphologisch unterschiedlichen Geweben. In der Haut befindet sie sich im wesentlichen als Blutgefäße umschließendes Gewebe und zwischen Dermis und Epidermis. Die letztgenannte Region trennt die Epidermis und ihre Anhanggebilde von der Dermis und wird entsprechend als Dermal-Epidermal-Junction (DEJ) bezeichnet. Die DEJ besitzt eine komplexe Struktur bestehend aus Hemidesmosomen, intermediären Filamenten, verankernden Filamenten, Lamina densa und verankernden Fibrillen. Zu den darin hauptsächlich vorkommenden biochemischen Komponenten zählen Laminin-5 in der Lamina lucida; die Antigene AgBP 230 und AgPB180 sowie Plectin/HD1 in Hemidesmosomen; Entactin/Nidogen und das Proteoglykan Perlecan in der Lamina lucida und Lamina densa; Collagen Typ IV in der Lamina densa und das Proteoglykan Collagen Typ VII als Bestandteil der verankernden Fibrillen in der sub-Lamina densa. Diese Komponenten bilden ein interagierendes Netzwerk.

Die DEJ stellt das wichtigste Gebilde der Haut dar. Sie sorgt für die Verbindung zwischen Epidermis und darunter liegender Dermis und erhält die Integrität des epithelialen Gewebes durch Verankerung von Zellen mit der extrazellulären Matrix über die speziellen verbindenden Zellkomplexe der Hemi-Desmosomen, Filamente und Fibrillen.

Einerseits stellt sie einen Filter für den Fluss spezieller Moleküle dar, andererseits ermöglicht sie den Austausch von Informationen - beispielsweise über Wachstumsfaktoren - zwischen Keratinocyten und Dermis.

Zu den alterungsbedingten Änderungen der DEJ zählen die abnehmende Dicke der Junctions und die Reduktion von den aus basalen Keratinocyten bestehenden cytoplasmatischen Zellzotten in der Dermis. Die dadurch resultierende Abnahme der Oberfläche der DEJ führt zu einem reduzierten Widerstand des Gewebes, einer Abnahme der Hautstraffung und einer vermehrten Bildung von Falten. Weitere Alterungserscheinungen wie Verdopplung der Lamina densa, Alterung der verankernden Fibrillen oder Modifikationen von Zellkomponenten der DEJ führen ebenfalls dazu, dass die Verankerung über das epidermale System mit zunehmendem Alter eine Lockerung erfährt. Die bei der Alterung bedingte Abnahme von Typ VII Collagen erklärt man sich auch durch die Hydrolyse des Collagens über Metalloproteinasen. Typ VII Collagen scheint mit zunehmendem Alter weniger resistent gegen Proteasen zu sein.

Auch durch UV-Strahlung induzierte Schädigungen, die zu einer Reduktion des Gehaltes an Typ VII Collagen in den verankernden Fibrillen führen, resultieren in einer Lockerung der Bindung zwischen Dermis und Epidermis und einer dadurch bedingten Abnahme der Hautelastizität und Zunahme von Falten.

Zahlreiche Krankheiten, die die DEJ beeinträchtigen, führen zu einer Ausbildung von subepidermalen Umfangsvermehrungen. Sie zeigen als gemeinsames Merkmal eine verminderte Cohesion zwischen Dermis und Epidermis, die sich auf der Ebene der DEJ in einer Bildung von Einbuchtungen manifestiert.

Mit dem Ziel einer Verbesserung der Funktion der Dermal Epidermal Junctions wurden insbesondere Bestandteile der DEJ wie Lamin (FR 2813018, WO 97/48415), oder Kalinin (WO 92/ 17498) in kosmetischen und dermatologischen Formulierungen eingesetzt. Auch die Stimulation von Typ IV Collagen durch Magnesiumaspartat (WO 99/62481), Saponine (FR 2779058) oder Pflanzenextrakte (EP 668072) sowie die Stimulation von Typ VII Collagen durch Elaginsäure (WO 99/16415), Pflanzenextrakt der Potentilla erecta (WO 98/19664) oder Bertholletia Extrakt (US 6004568) wurden offenbart zur Herstellung von kosmetischen Zubereitungen gegen Alterung, Faltenbildung und zur Straffung der Haut.

Dennoch besteht weiterer Bedarf an einem effektiven Schutz der Haut gegen umweltbedingte Alterungseinflüsse. Die Aufgabe der vorliegenden Patentanmeldung hat daher darin bestanden, neue Mechanismen zur Verbesserung der Dermal-Epidermal Junctions der Haut, Kopfhaut und Schleimhaut zu finden, die zu einer Verzögerung der Hautalterung und zu einem Schutz der Haut, Kopfhaut und Schleimhaut gegen Umwelteinflüsse, oxidativen Stress, toxische Substanzen oder UV-Strahlung beitragen und somit effektiv in kosmetischen Zubereitungen für die topische Anwendung genutzt werden können.

### Beschreibung der Erfindung

Gegenstand der Erfindung sind Verfahren zur kosmetischen Behandlung zur Verbesserung und/oder zum Schutz der Dermal-Epidermal Junctions der Haut, Kopfhaut und Schleimhaut, dadurch gekennzeichnet, dass man eine Zubereitung, enthaltend mindestens eine Substanz, welche eine Modulation von Plectin/HD1 und/oder Entactin/Nidogen und/oder Perlecan bewirkt, topisch aufträgt.

Weitere Gegenstände der Erfindung sind die Verwendung einer Substanz, welche eine Modulation von Plectin/HD1 und/oder Entactin/Nidogen und/oder Perlecan bewirkt, zur Herstellung kosmetischer Mittel zur Verbesserung und/oder zum Schutz der Dermal-Epidermal Junctions der Haut, Kopfhaut und Schleimhaut, die Verwendung dieser Substanz zur Herstellung kosmetischer Mittel zum Schutz gegen Hautalterung und die Verwendung der Substanz zur Herstellung kosmetischer Mittel zum Schutz gegen oxidativen Stress, schädigende Einflüsse durch Umweltgifte und UV-Strahlung.

Überraschenderweise wurde gefunden, dass die Modulation von Molekülen wie Plectin/HD1, Entactin/Nidogen und/oder Perlecan zu einer Erhaltung und Verbesserung der Dermal-Epidermal-Junctions der Haut, Kopfhaut und Schleimhaut führt. Die Funktion der DEJ ist eine essentielle Voraussetzung nicht nur für die Gesundheit, sondern auch für die Kosmetik. Sie sorgt für einen guten Zusammenhalt zwischen Epidermis und den darunter liegenden Geweben der Dermis, erhält somit die Elastizität und Straffheit der Haut und ermöglicht die Verhinderung von Faltenbildung. Darüber hinaus wird über die DEJ einerseits die gute Versorgung der Haut durch die Passage von lebensnotwendigen Molekülen zwischen Epidermis und Dermis gewährleistet, andererseits ein Schutz vor dem Eindringen schädigender Moleküle in tiefere Hautschichten geboten.

Die Modulation von Plectin/HD1, Entactin/Nidogen und/oder Perlecan über die topische Applikation einer Zubereitung, die diese Substanzen stimuliert, hat nun gezeigt, dass auf diese Weise die Dermal Epidermal Junction resp. das gesamte komplexe Netzwerk der DEJ gestärkt werden kann. Dieses hat eine Straffung der Haut und Verminderung der Faltenbildung zur Folge. Durch die verbesserte Verankerung zwischen den Komponenten der DEJ und die damit verbundene erhöhte Stabilität und zunehmende Elastizität des Gewebes können Alterungserscheinungen, auch wenn sie durch UV-Strahlung verursacht sind, effektiv vorgebeugt werden.

Bedingt durch die Verbesserung der Molekülpassagefunktion wird der Austausch zwischen Keratinocyten und Dermis und die Ernährung der Haut optimiert, und somit nicht nur die Haut gegen Alterung, sondern auch gegen schädigende Einflüsse von UV-Strahlung und toxischen Umwelteinflüssen geschützt, da durch die verbesserte Versorgung auch die Abwehr gegen schädigende Moleküle gestärkt wird.

Die DEJ umgibt auf der Kopfhaut die Haarfollikel und sorgt auch hier für einen Schutz der Follikel, so dass eine Stärkung der DEJ in diesem Bereich zu einer Verbesserung der Haareigenschaften führt und insbesondere wirksam ist gegen Haarausfall oder Haarschädigungen.

Somit haben die topisch applizierten kosmetischen Mittel, die mindestens eine Substanz enthalten, welche die Modulation von Plectin/HD1 und/oder Entactin/Nidogen und/oder Perlecan bewirkt, einen vorbeugenden Effekt gegen Hautalterung und schädigende Einflüsse durch oxidativen Stress, Umweltgifte und UV-Strahlung auf Haut, Kopfhaut, Haare und Schleimhaut. Die Modulation der speziellen Moleküle führt jedoch neben der vorbeugenden Wirkung auch zu einer beschleunigten Regeneration der Haut, Kopfhaut und Schleimhaut nach einer bereits eingetretenen Schädigung.

Als Modulatoren haben sich Pflanzenextrakte, insbesondere der Extrakt aus Pisum sativum, Ruscus Aculeatus, Centella asiatica, Calendula Officinalis, Aesculus Hippocastanum, und/oder Hibiscus esculentus als geeignet erwiesen. Möglich ist jedoch auch eine Modulation von Plectin/HD1 und/oder Entactin/Nidogen und/oder Perlecan über die Gabe von niedrigmolekularen Peptiden, die zum Aufbau der DEJ-Komponenten benötigt werden und eine ähnliche Sequenz aufweisen wie Bestandteile von Plectin/HD1 und/oder Entactin/Nidogen und/oder Perlecan.

Ebenfalls beobachtet wurde eine Modulation der Moleküle über mindestens eine Substanz, die ausgewählt ist aus der Gruppe, die gebildet wird von Mannitol, Cyclodextrin, Hefeextrakt, Panthenol, Propylen Glycol, Ammonium Glycyrrhizat und Dinatriumsuccinat. Insbesondere die Kombination dieser Bestandteile trägt zu einer vorteilhaften Wirkung auf die Dermal-Epidermal Junctions bei.

Da die DEJ für den Abbau durch eine Vielzahl unterschiedlicher Proteasen sehr empfänglich ist, trägt die Verwendung von Pflanzenextrakten, Peptiden und anderen Wirkstoffen mit Anti-Protease Aktivität entscheidend zum Erhalt der Struktur bei. Wirkstoffe mit Anti-Protease Aktivität können in Kombination mit Substanzen, welche die Modulation von Plectin/HD1 und/oder Entactin/Nidogen und/oder Perlecan bewirken, zu einer überdurchschnittlichen Wirkungssteigerung beitragen.

Neben diesen Substanzen oder Pflanzenextrakten können die kosmetischen Mittel außerdem UV-Lichtschutzfaktoren und/oder Antioxidantien enthalten. Die Kombination aus Substanzen, die eine Modulation von Plectin/HD1 und/oder Entactin/Nidogen und/oder Perlecan bewirken mit UV-Lichtschutzfaktoren und/oder Antioxidantien führt durch die unterschiedlichen Mechanismen zu einer synergistischen Wirkungsweise und bietet einen hervorragenden Schutz gegen schädigende Einflüsse und Hautalterung durch UV-Lichteinwirkung.

### Plectin/HD1

Plectin und HD1 sind Synonyme für das selbe Molekül. Es ist in den Hemi-Desmosomen lokalisiert, weist eine Molekülmasse von ca. 500 Dalton auf und dient der Verankerung von Proteinen des Cytoskellets wie Keratin, Vimentin oder Proteinen der Microtubuli.

### Entactin/Nidogen

Auch Entactin und Nidogen sind unterschiedliche Begriffe für das selbe Molekül. Es stellt ein Glycoprotein mit einer Molekülmasse von ungefähr 150 Dalton dar, das aus zwei terminalen globulären Regionen, die durch eine langkettige Struktur miteinander verbunden sind, besteht. Dieses Glycoprotein ist maßgeblich für die strukturelle Stabilität der DEJ verantwortlich, dadurch dass es eine Verbindung zwischen Laminin und Typ IV Collagen herstellt und zur Verankerung einer Vielzahl weiterer Komponenten wie Fibulin oder Perlecan beiträgt.

### Perlecan

Alle Basalmembranen enthalten Proteoglycane. Das hauptsächlich in der DEJ vorkommende Proteoglycan ist Perlecan, ein Heparansulfat, das von dermalen Fibroblasten synthetisiert wird. Perlecan besteht aus einem großen Proteinkern und drei Heparansulfatketten. Seine Aufgabe ist es unter anderem eine Bindung zwischen Laminin-6 und Nidogen zu stabilisieren. Neben den verankernden Funktionen binden Heparansulfat-Proteoglykane auch diffundierende Moleküle wie Enzyme und Wachstumsfaktoren und haben möglicherweise dadurch ebenfalls einen Einfluss auf das Verhalten und die Eigenschaften von Zellen.

### UV-Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
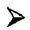 3-Benzylidencampher bzw. 3-Benzylidennorcampher und dessen Derivate, z.B. 3-(4-Methylbenzyliden)campher;
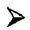 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
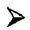 Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
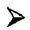 Ester der Salicylsäure, vorzugsweise Salicylsäure-2-ethylhexylester, Salicylsäure-4-isopropylbenzylester, Salicylsäurehomomenthylester;
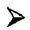 Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
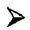 Ester der Benzalmalonsäure, vorzugsweise 4-Methoxybenzmalonsäuredi-2-ethylhexylester;
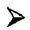 Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB);
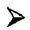 Propan-1,3-dione, wie z.B. 1-(4-tert.Butylphenyl)-3-(4'methoxyphenyl)propan-1,3-dion;
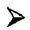 Ketotricyclo(5.2.1.0)decan-Derivate.

Als wasserlösliche Substanzen kommen in Frage:
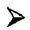 2-Phenylbenzimidazol-5-sulfonsäure und deren Alkali-, Erdalkali-, Ammonium-, Alkylammonium-, Alkanolammonium- und Glucammoniumsalze;
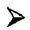 Sulfonsäurederivate von Benzophenonen, vorzugsweise 2-Hydroxy-4-methoxybenzophenon-5-sulfonsäure und ihre Salze;
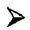 Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden deartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Beispiele

### Beispiel 1: Plectin Expression

### Reagentien:

Monoclonale Antikörper anti-plectin und secundäre Antikörper an Fluorescein Isothiocyanat FITC konjugierte anti-mouse Antikörper IgG wurden erhalten von der Firma Tebu und Clinisciences. PBS (Phosphat buffered saline pH 7,2) und Evans blau wurden von BioMérieux bezogen und TGF stammt von Sigma.

### EKIN rekonstruierte menschliche Haut:

Rekonstruierte menschliche Haut von Episkin (Ekin kit) besteht aus einer dermalen Stütze geformt aus Collagen. Die Keratinocyten wurden von dieser Stütze entfernt. Nach der Entfernung der Keratinocyten und einer Differenzierung in einer Luft-exponierten Kultur, konnte ein Äquivalent zur menschlichen Haut erhalten werden.

### Test Substanzen

TGF beta wurde als positiv-Kontrolle verwendet.
Als Testsubstanz diente eine Zubereitung bestehend aus: Propylen Glycol, Ruscus Aculeatus Wurzel Extrakt, Centella Asiatica Extrakt, Panthenol, Wasser, Calendula Officinalis Blumen Extrakt, hydrolysierte Hefeproteine, Aesculus Hippocastanum Axtralct, und Ammonium Glycyrrhizate in veränderlichen Gewichtsanteilen.
Die rekonstruierte menschliche Haut wurde täglich über drei Tage mit 0,01 % dieser Zubereitung oder mit 10 ng/ml TGF beta topisch behandelt. Dies entspricht einer Gesamtmenge an Zubereitung von 100 Mikroliter pro rekonstruierter Haut. Nach dieser Behandlung wurden umgehend Biopsien genommen und bis zur Auswertung in flüssigem Stickstoff gelagert.

### Immunohistochemie

Zehn Mikrometer der Kryostat Section (in flüssigem Stickstoff gelagerte Biopsie) wurden auf Objektträger aus Glas fixiert und in kaltem Aceton für zehn Minuten gelagert. Anschließend wurde die Section in PBS gewaschen und an der Luft getrocknet.
Die Section wurde eine Stunde bei Raumtemperatur mit dem monoclonalen Antikörper anti-plectin in einer Lösung von 1/150 inkubiert. Nach dem Waschen mit PBS wurde die Section mit Fluorescein Isothiocyanat (FITC) - konjugiertem anti-mouse Antikörper für 45 min in einer Lösung von 1/40 inkubiert. Negativ-Kontrollen wurden erhalten durch Weglassen des ersten Antikörpers. Nach dem ausgedehnten Waschen mit PBS wurden die immunobehandelten Sectionen für 10 min. mit Evans blau behandelt. Mit einem konvokalen Lasermikroskop der Firma Zeiss wurden die Sectionen begutachtet.

### Quantifizierung

Die durch das konvokale Lasermikroskop erhaltenen Bilder wurden konvertiert und analysiert durch eine mathematisch morphologische Software (Quantimet Q500, Leica). Die Ergebnisse wurden dargestellt als Prozent der rekonstruierten Hautoberfläche belegt durch Plectin (FITC) belegt wird.

### Ergebnisse

| | Kontrolle ohne Behandlung | TGF beta | Zubereitung der Testsubstanz |
|---|---|---|---|
| % des Plectin-Gehaltes in der Haut-Section | 4,16 | 21,43 | 37,9 |

Ohne Behandlung konnte nur eine geringe Expression der DEJ - Komponente Plectin in der rekonstruierten Haut nachgewiesen werden.
Die topische Behandlung mit der Zubereitung der Testsubstanzen haben eine starke Erhöhung der Expression von Plectin in der rekonstruierten Haut gezeigt. Die positiv-Kontrolle TGF zeigte ebenfalls eine Erhöhung der Expression an Plectin in der rekonstruierten Haut.

### Beispiel 2: Perlecan Expression

### Reagentien:

Monoclonale Antikörper Anti-perlecan und secundäre Antikörper an Fluorescein Isothiocyanat FITC konjugierte anti-mouse Antikörper IgG wurden erhalten von der Firma Tebu und Clinisciences. PBS (Phosphat buffered saline pH 7,2) und Evans blau wurden von BioMérieux bezogen und TGF stammt von Sigma.

### Menschliche primäre Fibroblasten Kultur

Eine Zellsuspension an menschlichen Fibroblasten wurde hergestellt durch Standard Verdauung von Collagenase von menschlicher Dermis erwachsener Personen, erhalten durch plastische Chirurgie. Die Fibroblasten wurden gezogen auf Glassschalen mit Inkubationskammern und wuchsen im Kulturmedium bis zum Zusammenfluss.

### Test Substanzen

TGF beta wurde als positiv-Kontrolle verwendet.
Als Testsubstanz diente ein hydrolysierter Hibiscus Esculentus Extrakt.
Die menschlichen Fibroblasten wurden kultiviert in Gegenwart von 0,1 % der Testsubstanz oder in Gegenwart von 10 ng/ml TGF beta in dem Kulturmedium über 6 Tage. Anschließend wurde die Perlecan Expression durch Immunocytochemie ausgewertet.

### Immunohistochemie

Die Fibroblasten Kultur in den Glassschalen wurden in kaltem Methanol für 10 min fixiert und mit PBS gewaschen. Anschließend wurden die Fibroblasten Kulturen eine Stunde bei 37°C mit dem monoclonalen Antikörper anti-plectin in einer Lösung von 1/150 inkubiert. Nach dem Waschen mit PBS wurde die Section mit Fluorescein Isothiocyanat (FITC) - konjugiertem anti-mouse Antikörper für 45 min in einer Lösung von 1/40 inkubiert. Negativ-Kontrollen wurden erhalten durch Weglassen des ersten Antikörpers. Nach dem ausgedehnten Waschen mit PBS wurden die immunobehandelten Sectionen für 10 min. mit Evans blau behandelt. Mit einem konvokalen Lasermilcroskop der Firma Zeiss wurden die Sectionen begutachtet.

### Quantifizierung

Die durch das konvokale Lasermikroskop erhaltenen Bilder wurden konvertiert und analysiert durch eine mathematisch morphologische Software (Quantimet Q500, Leica). Die Ergebnisse wurden dargestellt als Prozent der Fläche der Fibroblasten Kultur belegt durch Perlecan (FITC) belegt wird.

### Ergebnisse

| | Kontrolle ohne Behandlung | TGF beta | Testsubstanz |
|---|---|---|---|
| % des Perlecan Gehaltes in der Fibroblasten Kultur | 1,28 | 25,07 | 8,0 |

Ohne Behandlung konnte nur eine geringe Expression der DEJ - Komponente Perlecan in der Fibroblasten Kulturnachgewiesen werden.
Die Behandlung mit der Testsubstanz haben eine Erhöhung der Expression von Perlecanin in der Fibroblasten Kultur gezeigt. Die positiv-Kontrolle TGF zeigte ebenfalls eine Erhöhung der Expression an Perlecan in der Fibroblasten Kultur.

Die Ergebnisse aus Beispiel 1 und Beispiel 2 zeigen, dass die Testsubstanzen, (Produkte von Laboratoires Sérobiologiques) die Expression von Plectin und Perlecan erhöhen können.

## Patentansprüche

1. Verfahren zur kosmetischen Behandlung zur Verbesserung und/oder zum Schutz der Dermal-Epidermal-Junctions der Haut, Kopfhaut und Schleimhaut, **dadurch gekennzeichnet, dass** man eine Zubereitung, enthaltend mindestens eine Substanz, welche eine Modulation von Plectin/HD1 und/oder Entactin/Nidogen und/oder Perlecan bewirkt, topisch aufträgt,
wobei die Substanz ausgewählt wird aus der Gruppe bestehend aus einem Extrakt aus der Pflanze Pisum sativum, Ruscus Aculeatus, Centella asiatica, Calendula Officinalis, Aesculus Hippocastanum, und/oder Hibiscus esculentus und Mannitol, Cyclodextrin, Hefeextrakt, Panthenol, Propylen Glycol, Ammoniumglycyrrhizat, Dinatriumsuccinat und niedrigmolekularen Peptiden.

2. Kosmetische Verwendung einer Substanz wie definiert in Anspruch 1 zum Schutz der Haut gegen Alterung.

3. Kosmetische Verwendung einer Substanz wie definiert in Anspruch 1 zum Schutz der Haut, Kopfhaut und Schleimhaut gegen toxische Umwelteinflüsse.

4. Kosmetische Verwendung einer Substanz wie definiert in Anspruch 1 zum Schutz der Haut, Kopfhaut und Schleimhaut gegen UV-Lichteinwirkung.

5. Kosmetische Verwendung einer Substanz wie definiert in Anspruch 1 gegen oxidativen Stress.

6. Kosmetische Verwendung einer Substanz wie definiert in Anspruch 1 gegen Haarausfall und zur Verbesserung der Haareigenschaften.

7. Kosmetische Zubereitungen, enthaltend mindestens eine Substanz wie definiert in Anspruch 1, welche eine Modulation von Plectin/HD1 und/oder Entactin/Nidogen und/oder Perlecan bewirkt, und UV-Lichtschutzfaktoren und/oder Antioxidantien.

## Claims

1. A method for cosmetic treatment for improving and/or protecting the dermal-epidermal junctions of the skin, scalp and mucous membranes, wherein a preparation comprising at least one substance which modulates plectin/HD1 and/or entactin/nidogen and/or perlecan is topically applied,
wherein the substance is selected from the group consisting of an extract of the plant Pisum sativum, Ruscus aculeatus, Centella asiatica, Calendula officinalis, Aesculus hippocastanum and/or Hibiscus esculentus and mannitol, cyclodextrin, yeast extract, panthenol, propylene glycol, ammonium glycyrrhizate, disodium succinate and low molecular weight peptides.

2. The cosmetic use of a substance as defined in claim 1 for protecting skin against aging.

3. The cosmetic use of a substance as defined in claim 1 for protecting skin, scalp and mucous membranes from toxic environmental influences.

4. The cosmetic use of a substance as defined in claim 1 for protecting skin, scalp and mucous membranes from the effects of UV light.

5. The cosmetic use of a substance as defined in claim 1 against oxidative stress.

6. The cosmetic use of a substance as defined in claim 1 against hair loss and to improve hair properties.

7. A cosmetic preparation comprising at least one substance as defined in claim 1, which modulates plectin/HD1 and/or entactin/nidogen and/or perlecan, and UV light protection factors and/or antioxidants.

## Revendications

1. Procédé pour le traitement cosmétique en vue de l'amélioration et/ou de la protection de la jonction derme-épiderme de la peau, du cuir chevelu et de la muqueuse, **caractérisé en ce qu'**on applique une composition, contenant au moins une substance qui provoque une modulation de la plectine/HD1 et/ou de l'entactine/nidogène et/ou du perlécane, par voie topique, la substance étant choisie dans le groupe constitué par un extrait des plantes Pisum sativum, Ruscus Aculeatus, Centella asiatica, Calendula Officinalis, Aesculus Hippocastanum et/ou Hibiscus esculentus, et du mannitol, de la cyclodextrine, de l'extrait de levure, du panthénol, du propylèneglycol, du glycyrrhizate d'ammonium, du succinate disodique et des peptides de bas poids moléculaire.

2. Utilisation cosmétique d'une substance telle que définie dans la revendication 1 pour la protection de la peau contre le vieillissement.

3. Utilisation cosmétique d'une substance telle que définie dans la revendication 1 pour la protection de la peau, du cuir chevelu et de la muqueuse contre des influences environnementales toxiques.

4. Utilisation cosmétique d'une substance telle que définie dans la revendication 1 pour la protection de la peau, du cuir chevelu et de la muqueuse contre l'effet de la lumière UV.

5. Utilisation cosmétique d'une substance telle que définie dans la revendication 1 contre le stress oxydatif.

6. Utilisation cosmétique d'une substance telle que définie dans la revendication 1 contre la chute des cheveux et pour l'amélioration des propriétés des cheveux.

7. Préparations cosmétiques, contenant au moins une substance telle que définie dans la revendication 1, qui provoque une modulation de la plectine/HD1 et/ou de l'entactine/nidogène et/ou du perlécane, et des facteurs de protection contre la lumière UV et/ou des antioxydants.
